# EUROPEAN PATENT APPLICATION

(11) **EP 2 609 867 A1**
(43) Date of publication of application: **03.07.2013**
(21) Application number: 12199483.4
(22) Date of filing: 27.12.2012
(51) Int. Cl.: A61B 8/00

(54) **Ultrasound diagnostic apparatus and method of controlling the same**

(30) Priority: 26.12.2011 KR 20110142451
(71) Applicant: Samsung Medison Co., Ltd., Gangwon-do 250-870 (KR)
(72) Inventor: Kim, Jung Bae, Seoul (KR)
(74) Representative: Lorenz, Markus

(57) **Abstract**

An ultrasound diagnostic apparatus and a method of controlling the same are provided herein. The ultrasound diagnostic apparatus includes a probe having a connector, provided with a shaft, and a probing part, the probing part being connected to the connector through a cable so as to irradiate ultrasound waves to an object to be examined and receive the ultrasound waves reflected from the object, a main body connected with the probe so as to create an image of the object, which corresponds to the ultrasound waves received to the probing part of the probe, a coupling assembly to detect contact between the main body and the connector and to fix the connector to the main body by automatically driving the shaft of the probe, and a controller to control driving of the coupling assembly when the contact between the main body and the connector is detected.

## Description

### TECHNICAL FIELD

Embodiments of the present disclosure relate to an ultrasound diagnostic apparatus to facilitate fastening between a main body and a probe, and a method for controlling the same.

### BACKGROUND

In general, an ultrasound diagnostic apparatus generates an image of an object to be examined from a reflected ultrasound signal, which has been irradiated into the object using probes. Such an ultrasound diagnostic apparatus has been particularly useful for various medical diagnostic procedures including, for example, the detection of foreign matter in living things, measurement of lesions, observation of tumors, examination of fetuses, etc.

An ultrasound diagnostic apparatus typically includes a main body equipped with an input device to receive input from a user, a display device to generate an image of an object to be examined, and various types of probes to irradiate an ultrasound signal into the object and to receive an ultrasound echo signal therefrom. For example, three or four probes may be coupled to the main body. Each probe may be locked into the main body of the ultrasound diagnostic apparatus using a specialized locking mechanism according to the characteristics of the particular probe. Furthermore, each of the probes may be assigned an intrinsic identification number or identifier (ID), which can be used by a medical doctor to identify and select a probe to be used. A user such as a medical doctor can manipulate an input device of the ultrasound diagnostic apparatus to identify and select a particular probe for use based on its corresponding identification number or ID.

Each probe typically includes a probing part having an array of ultrasound elements, a connector to electrically connect each of the ultrasound elements of the probe to the main body of the apparatus, and a cable to connect the probing part to the connector. The number of ultrasound elements of the probing part typically varies from 64 to 256 elements, and each probe includes the same number of connectors as the number of ultrasound elements.

The connector of each probe includes a housing, a terminal having a plurality of pins aligned within the housing, a shaft formed to outwardly protrude from an inner portion of the housing so as to mechanically connect the connector to the main body, and a locking member formed to protrude from an outer peripheral surface of the shaft so as to mechanically lock the connector into the main body. The connector further includes a locking handle arranged at an outer portion of the housing and mechanically connected to the shaft so as to rotate the shaft for locking between the connector and the main body.

In order to connect each probe to the main body, a user inserts the terminal of the connector of the probe into a socket of the main body, and couples the locking member of the shaft to a locking groove within the main body by manually turning the locking handle, thereby locking the probe in the main body. In addition, the rotation of the locking handle causes the pins of the terminal of the connector to be moved from their present positions and come into contact with corresponding pins of the socket of the main body, thereby electrically connecting the terminal to the main body.

When desiring to utilize a different type of probe from the probe which is presently in use, a user separates the connector of the present probe from the main body and then mounts the connector of a desired probe to the main body. The user removes the present probe from the main body by manually turning the locking handle of the present probe. The user can then mount another probe at the main body by manually turning the locking handle to lock the probe in place after connecting it to the main body.

Due to the manual nature of this task, the replacement of different probes may be cumbersome and may lead to a breakdown of the locking member and locking handle over time. Furthermore, since each probe has a structure in which a shaft of each of its connectors outwardly passes through an inner portion of the housing, the installation of printed circuit boards (PCBs) within the housing of the connector of the probe may be difficult and thus lead to problems in manufacturing the PCBs.

### SUMMARY

Therefore, it is an aspect of the present disclosure to provide an ultrasound diagnostic apparatus and method for detecting contact between a probe and a main body of the ultrasound diagnostic apparatus and automatically locking the probe into the main body when the contact between the probe and the main body is detected.

It is another aspect of the present disclosure to provide an ultrasound diagnostic apparatus having probe and main body components that are connected to one another primarily through a mechanical connection and secondarily, through an electrical connection, and a method of controlling the same.

It is a further aspect of the present disclosure to provide an ultrasound diagnostic apparatus to automatically release a lock between a probe and a main body of the apparatus in response to a release command input by a user.

Additional aspects of the present disclosure will be set forth in the detailed description that follows or will become readily apparent given this description.

In accordance with one aspect of the present disclosure, an ultrasound diagnostic apparatus includes a probe having a connector, provided with a shaft, and a probing part connected to the connector through a cable. The probing part is used to irradiate ultrasound waves through an object to be examined and to receive the ultrasound waves reflected from the object. The ultrasound diagnostic apparatus also includes a main body connected to the probe. The main body is configured to generate an image of the object, e.g., on a display coupled to the main body, based on the ultrasound waves received from the probing part of the probe. The ultrasound diagnostic apparatus further includes a coupling assembly configured to detect contact between the main body and the connector of the probe and to fix the connector to the main body by automatically driving the shaft of the probe. A controller of the ultrasound diagnostic apparatus is used to control driving of the coupling assembly when the contact between the main body and the connector is detected.

The coupling assembly may include a coupling member coupled to the shaft, a motor to rotate the coupling member, a locking groove to seat a locking member arranged at the shaft during rotation of the shaft, and a detection part to detect the contact between the main body and the connector.

The detection part may include at least one of an infrared detection part, a radio frequency (RF) detection part, a switch part, and a pressure detection part.

The controller may control rotation of the motor in a normal direction so that the locking member is seated at the locking groove, when the contact between the main body and the connector is detected.

The ultrasound diagnostic apparatus may further include an input part used to input a command by a user, wherein the controller may be configured to control rotation of the motor in a reverse direction so that the locking member is separated from the locking groove based on the command input by the user via the input part.

The ultrasound diagnostic apparatus may further include a fixing member to mechanically fix the probe to the main body.

The fixing member may be an electromagnet.

The controller, when the contact between the main body and the connector is detected, may be configured to allow the current to be applied to the electromagnet. In response to the comment input by the user via the input part, the controller may be further configured to block the current applied to the electromagnet so that the probe is separated from the main body.

The fixing member may be a mechanical hook, and responsive to the command input by the user via the input part, the controller of the ultrasound diagnostic apparatus may be further configured to control movement of the hook so that the probe is separated from the main body.

The cross section of the shaft of the probe may have a polygonal shape or an oval shape, and the coupling member may have a coupling groove formed in a corresponding shape within the shaft.

The connector may include a housing, a printed circuit board arranged within the housing to drive the probing part, a terminal having a plurality of pins electrically connected to the printed circuit board, and the shaft formed to outwardly protrude from an inner portion of the housing so as to move positions of the pins in the terminal.

The controller may control driving of the shaft so that the positions of the pins in the connector are moved to establish an electrical connection between the main body and the probe.

In accordance with another aspect of the present disclosure, a method for controlling an ultrasound diagnostic apparatus enables a probe to be connected to and separated from a main body of the apparatus through a connector of the probe. The method includes detecting contact between the main body and the connector through a detection part, automatically rotating a shaft arranged at the probe using a motor when the contact is detected, determining locking between the main body and the connector, and stopping the motor when the locking between the main body and the connector is completed.

A method for controlling the ultrasound diagnostic apparatus may further include electrically connecting the connector to the main body through the locking after mechanically fixing the connector to the main body through a fixing member, when the contact between the main body and the connector is detected.

A method for controlling the ultrasound diagnostic apparatus may further include determining whether or not a command is input via an input part, and when the command is input via the input part, controlling driving of the motor to release the locking between the main body and the connector.

A method for controlling the ultrasound diagnostic apparatus may further include determining whether or not a command is input via an input part, and when the command is input via the input part, controlling the fixing member to mechanically separate the connector from the main body.

The determination of whether or not the main body is locked to the connector may include detecting a current of the motor, and determining that the connector is locked to the main body when the current of the motor is at least a predetermined current.

Additionally or alternatively, the determination of whether or not the main body is locked to the connector may include detecting a number of rotations of the motor, and determining that the connector is locked into the main body when the number of rotations of the motor is equal to a predetermined minimum number of rotations.

In accordance with a further aspect of the present disclosure, an ultrasound diagnostic apparatus includes a probe having a connector, provided with a shaft, and a probing part. The probing part is connected to the connector through a cable so as to irradiate ultrasound waves to an object to be examined and receive the ultrasound waves reflected from the object. A main body is connected with the probe so as to create an image of the object, which corresponds to the ultrasound waves received by the probing part of the probe. A coupling assembly is used to input a command by a user and to fix the connector to the main body by automatically driving the shaft of the probe. A controller controls the driving of the coupling assembly based on the command that is input by the user.

The coupling assembly may include a coupling member coupled to the shaft, a motor to rotate the coupling member, a locking groove to seat a locking member arranged at the shaft during rotation of the shaft, and an input part used to input a command by a user. When a command is input by the user, the controller may control rotation of the motor in a normal direction so that the locking member is seated at the locking groove to lock the connector into the main body. However, when the command is input in a locked state, the controller may control rotation of the motor in a reverse direction to release the locking between the connector and the main body.

In accordance with yet another aspect of the present disclosure, an ultrasound diagnostic apparatus includes a probe having a connector, provided with a shaft, and a probing part. The probing part is connected to the connector through a cable and is used to irradiate ultrasound waves into an object to be examined and also, to receive the ultrasound waves reflected from the object. A main body is connected to the probe, which creates an image of the object based on the ultrasound waves received by the probing part of the probe. A fixing member mechanically fixes the connector to the main body. A coupling assembly locks the connector to the main body by automatically driving the shaft of the probe. A controller controls the driving of the coupling assembly when the mechanical contact between the connector and the main body is detected.

The fixing member may include an electromagnet, the controller may determine magnetic field change of the electromagnet, and when the magnetic field is changed, the controller may control rotation of the motor in a normal direction so as to electrically connect the connector to the main body.

The fixing member may include a hook. The controller may detect movement of the hook, and when movement of the hook is detected, rotate the motor in a normal direction so as to electrically connect the connector to the main body.

The ultrasound diagnostic apparatus may further include an input part used to receive a command input by a user. The controller may control the fixing member so that the connector is separated from the main body, based on the command input by the user. The controller may also control the rotation of the motor so that it rotates in a reverse direction and releases the locking between the connector and the main body.

In accordance with yet another aspect of the present disclosure, an ultrasound diagnostic apparatus includes a probe having a connector, provided with a shaft hole, and a probing part. The probing part is connected to the connector through a cable and used to irradiate ultrasound waves into an object to be examined and also, to receive the ultrasound waves reflected from the object. A main body is connected to the probe, which creates an image of the object based on the ultrasound waves received by the probing part of the probe. A coupling assembly detects contact between the main body and the connector and drives a shaft of the probe. A controller controls protrusion and rotation of the shaft so that the shaft is coupled to the shaft hole of the connector, when the contact between the main body and the connector is detected.

The ultrasound diagnostic apparatus may further include an input part configured to receive input, such as a command, from a user. The controller may control the rotation and retraction of the shaft so that the shaft is separated from the shaft hole, in accordance with the command received from the user.

The coupling assembly may include a protrusion member to protrude the shaft, and a motor to rotate the shaft.

The shaft may electrically connect the connector to the main body.

Additional advantages and novel features will be set forth in part in the description which follows, and in part will become apparent to those skilled in the art upon examination of the following and the accompanying drawings or may be learned by production or operation of the examples. The advantages of the present teachings may be realized and attained by practice or use of various aspects of the methodologies, instrumentalities and combinations set forth in the detailed examples discussed below.

### BRIEF DESCRIPTION OF THE DRAWINGS

These and/or other aspects of the present disclosure will become apparent and more readily appreciated from the following description of the embodiments, taken in conjunction with the accompanying drawings of which:
FIG. 1 illustrates a view of an exemplary ultrasound diagnostic apparatus;
FIG. 2 is an exemplary view illustrating an outer connection portion between a main body and one probe of the ultrasound diagnostic apparatus;
FIGS. 3 and 4 are exemplary views illustrating an inner connection portion between the main body and the probe of the ultrasound diagnostic apparatus;
FIGS. 5 to 8 are exemplary views illustrating a fixing member arranged at the ultrasound diagnostic apparatus;
FIG. 9 is a process flowchart of an exemplary ultrasound diagnostic apparatus;
FIG. 10 is a view illustrating an exemplary ultrasound diagnostic apparatus;
FIGS. 11 and 12 are views illustrating another exemplary ultrasound diagnostic apparatus; and
FIGS. 13 and 14 are views illustrating yet another exemplary ultrasound diagnostic apparatus.

### DETAILED DESCRIPTION

Reference will now be made in detail to the examples illustrating various aspects of the present disclosure, which are illustrated in the accompanying drawings, wherein like reference numerals generally refer to like elements throughout.

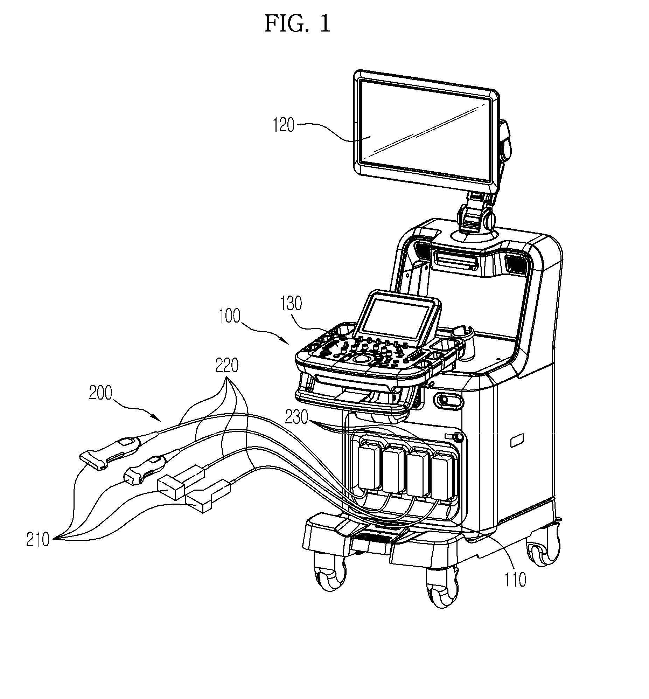

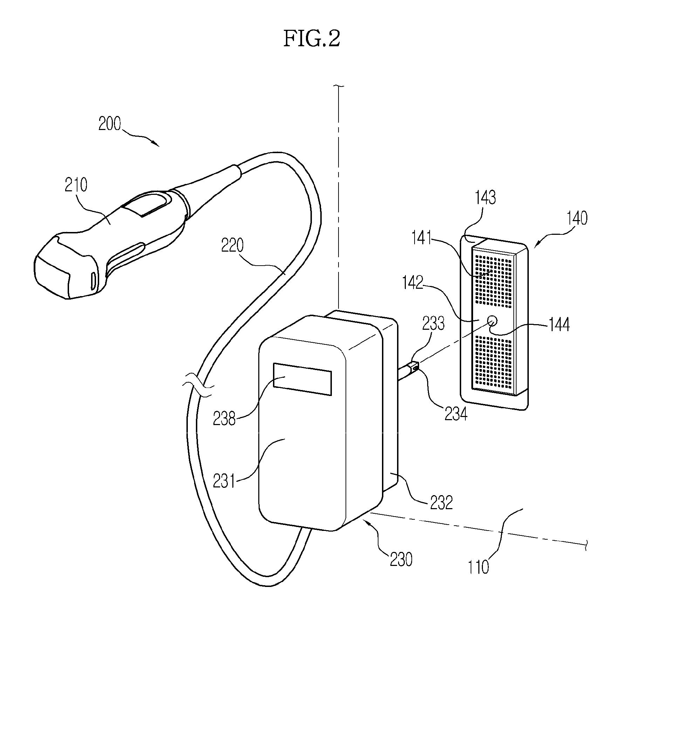

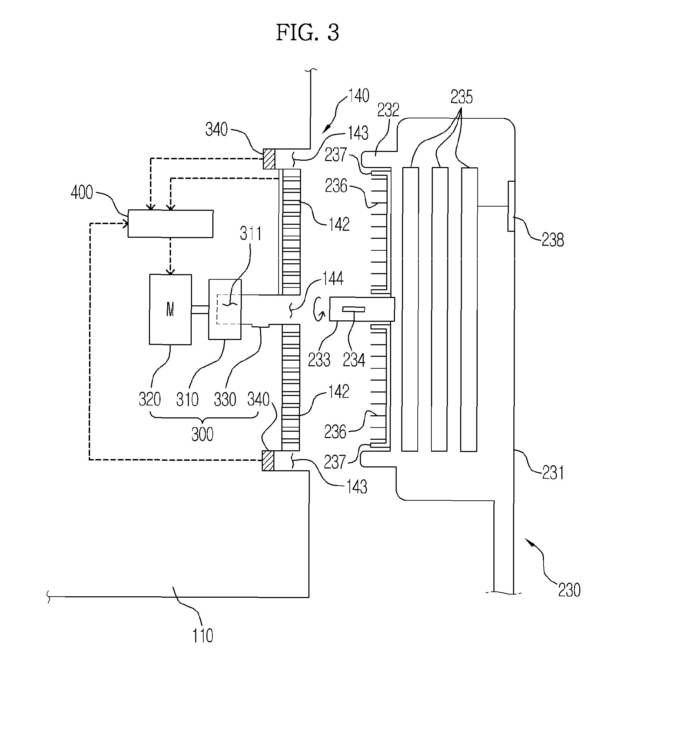

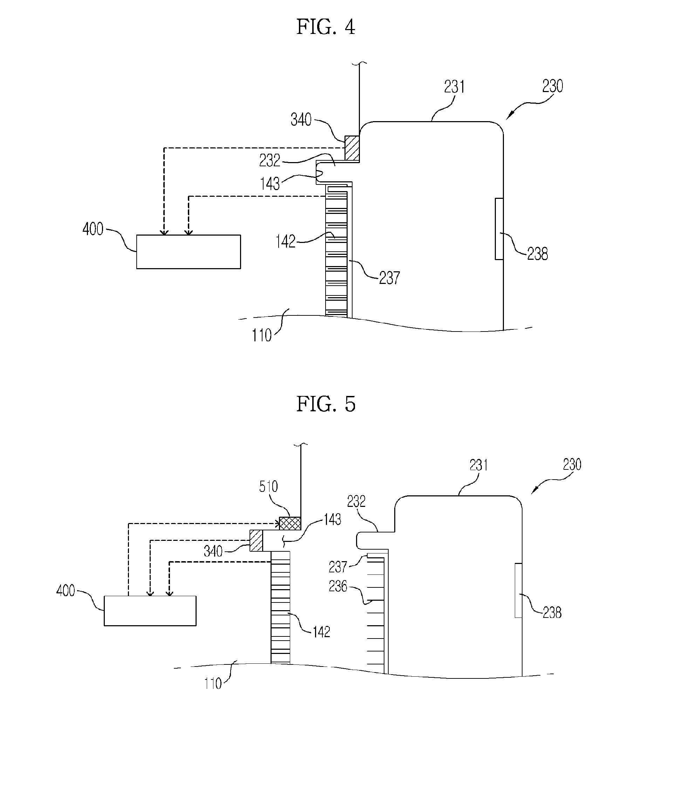

FIG. 1 illustrates a view of an exemplary ultrasound diagnostic apparatus. FIG. 2 is an exemplary view illustrating an outer connection portion between a main body and one probe of the exemplary ultrasound diagnostic apparatus of FIG. 1. FIGS. 3 and 4 illustrate views of an inner connection portion between the main body and the probe of the exemplary ultrasound diagnostic apparatus of FIG. 1. As shown in FIG. 1, the ultrasound diagnostic apparatus includes a main body 100, probes 200, a coupling assembly 300, and a controller 400.

The main body 100 includes a body part 110, a display part 120 to display a result of an ultrasound of an object being examined, e.g., as part of a diagnostic procedure being performed. The main body 100 further includes a manipulation part 130 provided with manipulation buttons to operate the probes 200 as well as the display part 120 and other component parts of the ultrasound diagnostic apparatus. The main body 100 may further include a control unit (not shown) arranged within the body part 110 so as to control the probes 200, the display part 120, and other components based on a manipulation result of the manipulation part 130. As shown in FIG. 2, and as will be described in further detail below , a socket part 140 is arranged within the body part 110 so as to mechanically and electrically connect each probe 200 to the main body 100.

In an example, the above-described control unit of the main body 100 converts signals received from the probes 200 into analog/digital receive signals. This may include, for example, properly executing time delays of the converted receive signals and summing the time delayed receive signals. This enables the output of digital receive-focused beams, which are signals exhibiting energy levels, which are reflected from focused points on respective transmit scan lines.

In order to improve the picture quality of an ultrasound image, the control unit filters out a noise ingredient in each digital receive-focused beam, and may also perform envelope detection processing in order to detect the intensity of a receive signal based on the filtered digital receive-focused beam, thereby producing digital ultrasound image data. Subsequently, the control unit performs scan conversion to convert a scan line of the digital ultrasound image data so that the digital ultrasound image data may be displayed on a display region of the display part 120. The control unit may perform additional image processing including, for example and without limitation, B-mode image processing, Doppler image processing, or the like, of the scan-converted digital ultrasound image data in order for it to be displayed as an ultrasound image on the display part 120 and as desired by a user.

Thereafter, the control unit executes red-green-blue (RGB) image processing of the image-processed digital ultrasound image, and transmits the RGB-processed digital ultrasound image data to the display part 120 so that the digital ultrasound image data may be displayed in the form of an ultrasound image on the display part 120. Thus, the digital ultrasound image data received by the display part 120 is converted into the ultrasound image and displayed on a screen of the display part 120.

The manipulation part 130 may be used by a user to input a diagnostic date, information of an object to be examined, and the like. The manipulation part 130 also may be used to select a particular region of the ultrasound image to be displayed on the display part 120.

The main body 100 further includes a storage part (not shown), which compresses the digital ultrasound image data using, for example, a predetermined compression technique and stores the compressed digital ultrasound image data in association with other relevant information including, but not limited to, the diagnostic date, information pertaining to the object being examined, and the any other information provided by the user and received as input through the manipulation part 130.

The storage part may store all of the digital ultrasound image data for an object. Alternatively, the storage may include only a portion of the digital ultrasound image data corresponding to a partial region of the object being examined, for example, as selected by a user using the manipulation part 130 from the ultrasound image displayed on the display part 120.

As shown in FIG. 2, the socket part 140 includes a socket 142 having socket grooves 141 into which connection pins of a connector 230 of the associated probe 200 are inserted during connection of the connector 230 of the probe 200, a seating groove 143 formed at an outer peripheral surface of the socket 142, to seat the connector 230 of the probe 200, and an insertion hole 144 formed at a predetermined region of the socket 142, to insert a shaft 233 of the connector 230 of the probe 200.

Here, the socket 142 further includes socket pins (not shown), to which the respective connection pins of the connector 230 inserted into the socket grooves 141 are connected. The socket pins electrically connect the connector 230 to the control unit (not shown) of the main body 100 (shown in FIG. 1), thereby allowing a signal of the associated probe 200 to be transmitted to the control unit.

That is, when the connector 230 of the probe 200 is connected to the socket part 140 of the main body 100, a probing part 210 of the probe 200 is electrically connected to various components of the main body 100. Thus, the main body 100 may control various components of the probe 200, thereby activating the probe 200. In addition, the main body 100 may process or display various information acquired by the probe 200.

One or more socket parts 140 may be provided, and the probes 200 having characteristics that vary from one another may be selectively connected thereto the plurality of socket parts 140.

Each probe 200 may be a separate component of the ultrasound diagnostic apparatus to directly execute an ultrasound diagnostic procedure for a portion of an object to be examined. The probe 200 is connected to the main body 100 and may execute ultrasound diagnostics based on a command transmitted from the main body 100. The probe 200 may then transmit a diagnostic ultrasound signal to the main body 100.

Such a probe 200 may be formed in various shapes and have an intrinsic characteristic. Each of the plurality of probes 200 may have intrinsic characteristics that vary from one another. Therefore, the probes 200 may be selectively connected to the main body 100 depending on the particular type and purpose of an examination to be conducted.

In an example, the probes 200 are mechanically and electrically connected to the main body 100. As shown in the example of FIG. 2, each probe 200 includes a probing part 210 having a plurality of ultrasound elements, for example, arranged in an array formation. The probing part 210 is used to directly perform ultrasound diagnosis of an object to be examined using the ultrasound elements. Each probe 200 includes a cable 220 connected at one side thereof to the probing part 210 and a connector 230, which is connected to the other side of the cable 220. The connector 230 has connection pins corresponding in number to the number of the ultrasound elements.

The probing part 210 of each probe 200 includes a one-dimensional, two-dimensional, or three-dimensional transducer (not shown).

The probing parts 210 of the respective probes 200 transmit ultrasound beams, including pulse signals that are properly delayed and produced by the respective transducers, to an object to be examined along transmit scan lines. The probing parts 210 also convert the ultrasound signals (or ultrasound echo signals) reflected from the object into electrical signals, each signal having a different time of receipt with respect to the respective transducers. The converted electrical signals are transmitted from the probes 200 to the main body 100.

The connector 230 of each probe 200 is electrically connected to the probing part 210 through the cable 220, and thus an electrical signal of the probing part 210 is transmitted to the connector 230 through the cable 220. The electrical signal is transmitted to the control unit of the main body 100.

As shown in FIG. 2, the connector 230 includes, for example and without limitation, a housing 231, a seating member 232 formed to protrude from a flange portion of one surface of the housing 231, a shaft 233 formed to protrude outwardly from an inner portion of the housing 231 so as to mechanically connect the connector 230 to the socket part 140 of the main body 100, and a locking member 234 configured to maintain an electrical connection between the connector 230 and the socket part 140 as well as to mechanically lock the connector 230.

The seating member 232 of the connector 230 may be, for example, a ferromagnetic substance made of a metal capable of being magnetized in a magnetic field. Such a metal may have, for example, strong magnetic properties and may include, but is not limited to, iron (Fe), nickel (Ni), or cobalt (Co), or an alloy thereof.

The shaft 233 has a polygonal shape or an oval shape in section so as to be stably locked into a coupling member 310, as illustrated in the example of FIG. 3.

As shown in FIG. 3, the connector 230 further includes at least one printed circuit board (PCB) 235 to enable a control signal and a diagnostic signal to be exchanged between the main body 100 and the associated probe 200. The control signal of the main body 100 is also used to drive the probe(s) 200 and connection pins 236 electrically connected to at least one PCB 235. The connection pins 236 are repositioned depending on the rotation of the shaft 233 so as to electrically connect the probe 200 to the main body 100. A terminal 237 includes a plurality of connection pins 236, which are inserted into the socket 142 of the socket part 140. The connector 230 also includes an input part 238 configured to receive input, including commands, from a user.

The input part 238 may include hardware buttons or the like for user control, which are electrically connected to at least one PCB 235. For example, when a user presses a button of the input part 238, a signal is generated by the input part 238 and transmitted to the PCB 235 electrically connected to the input part 238.

In this case, the PCB 235 electrically connected to the input part 238 transmits the signal of the input part 238 to the main body 100 through any one of the connection pins 236. Thus, the connector 230 is mechanically and electrically separated from the main body 100.

Alternatively, the input part 238 may be arranged at the main body 100.

In order to enhance user convenience, the socket part 140 of the main body 100 should be mechanically and electrically coupled to the connector 230 of the probe 200 so as to allow coupling to each other to be secure and easy.

To this end, the ultrasound diagnostic apparatus further includes a coupling assembly 300 and a controller 400.

The coupling assembly 300 and the controller 400 may be provided within the body part 110 of the main body 100 or within the housing 231 of the connector 230 of the probe 200. In the example illustrated in FIG. 3, the coupling assembly 300 and the controller 400 are arranged within the body part 110 of the main body 100.

Also, as shown in FIG. 3, the coupling assembly 300 includes a coupling member 310 coupled to an end of the shaft 233 of the connector 230 of the associated probe 200 and rotated by interlocking with the shaft 233 while being disposed at a position extending from the insertion hole 144 of the socket part 140, a motor 320 to apply rotation force to the coupling member 310 so as to drive the shaft 233, a locking groove 330 to seat the locking member 234 of the shaft 233, and a detection part 340 to detect contact between the socket part 140 and the connector 230.

On the other hand, the coupling member 310 further includes a coupling groove 311 to which the end of the shaft 233 is inserted. The coupling groove 311 has a shape that matches the sectional shape of the shaft 233, and the locking groove 330 serves to lock the connector 230 into the main body 100 during seating of the locking member 234.

As such, it may be possible to prevent the inserted probe 200 from being disconnected from the main body 100, for example, when the probe 200 is pulled excessively while a user diagnoses an object using the probe 200. Furthermore, it may also be possible to prevent the connection pins 236 from being bent due to the application of excessive force to the connector 230.

Further, as shown in FIG. 3, the detection part 340 is located within the seating groove 143. In this example, when the seating member 232 of the connector 230 comes into contact with the seating groove 143, for example, when the seating member 232 is inserted into the seating groove 143, the detection part 340 detects contact between the seating member 232 and the seating groove 143.

As shown in FIG. 4, the detection part 340 may also be located at a flange portion of the seating groove 143 of the body part 110. In this case, when the housing 231 of the connector 230 comes into contact with the body part 110 as the seating member 232 of the connector 230 is inserted into the seating groove 143, the detection part 340 may detect contact between the housing 231 and the body part 110.

Such a detection part 340 includes at least one infrared detection part to transmit infrared light and to detect the transmitted infrared light. The detection part 340 may further include, for example and without limitation, a radio frequency (RF) detection part to transmit radio frequencies and to detect the transmitted radio frequencies, an ultrasound detection part to generate ultrasound waves and to detect the reflected ultrasound waves, a noncontact detection part such as a capacitance detection part to detect capacitance or the like, a pressure detection part to detect pressure corresponding to contact of the connector 230, and a contact detection part such as a switch part turning ON during contact of the connector or the like.

Here, each of the infrared detection part, the RF detection part, and the ultrasound detection part include a part for transmitting RF signals and a part for receiving such signals. Referring back to FIG. 3, the RF transmitter part and RF receiver part may be arranged at upper and lower portions or left and right portions of the socket part 140, respectively.

The controller 400 is electrically connected to the motor 320. The controller 400 transmits a drive control signal to the motor 320 so as to execute automatic locking between the main body 100 and the connector 230 when contact between the main body 100 and the connector 230 is detected through the detection part 340. On the other hand, the controller 400 transmits a drive control signal to the motor 320 so as to automatically release locking between the main body 100 and the connector 230 when the signal is transmitted from the input part 238.

Here, locking between the main body 100 and the connector 230 refers to a case in which the locking member 234 of the shaft 233 is seated at the locking groove 330 of the coupling assembly 300 by appropriately rotating the shaft 233. As such, the connector 230 may be mechanically fixed to the main body 100 while the connection pins 236 of the connector 230 simultaneously come into contact with the socket pins of the socket part 140. As a result, the connector 230 is both mechanically and electrically connected to the main body 100.

The ultrasound diagnostic apparatus in the illustrated embodiment further includes a fixing member capable of enhancing connection force between the connector 230 of the associated probe 200 and the socket part 140 of the main body 100.

The fixing member may be provided in the main body 100 or the connector 230 of the probe 200. Hence, while the examples provided herein are describe the fixing member as being arranged at the main body 100, the ultrasound diagnostic apparatus and techniques described herein are not intended to be limited thereto.

The following description will be given with reference to FIGS. 5 to 8.

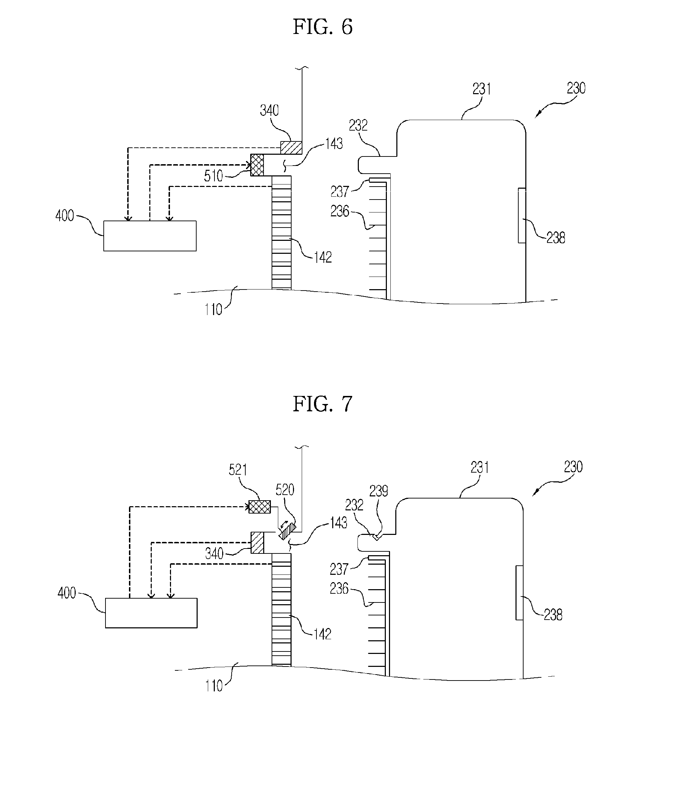

As shown in FIGS. 5 and 6, the fixing member includes an electromagnet 510 to generate electromagnetic force using applied current.

Current is applied to or blocked from the electromagnet 510, which refers to the fixing member, depending on a control command of the controller 400.

In more detail, current is applied to the electromagnet 510 to generate electromagnetic force when the connector 230 is coupled to the main body 100, whereas the current is blocked from the electromagnet 510 so as to remove the electromagnetic force when the connector 230 is mechanically separated from the main body 100.

That is, when the main body 100 comes into contact with the connector 230 through the detection part 340, the controller 400 allows current to be applied to the electromagnet 510, whereas when a command is input via the input part 238, the controller 400 blocks the current from being applied to the electromagnet 510.

In some implementations, the controller 400 may be configured to block the electromagnet 510 only for a predetermined period of time, for example, in accordance with a command input via the input part 238. The predetermined time period may start, for example, when the command is input by the user or received by the input part 238.

The electromagnet 510 may be arranged at a position to seat the seating member 232, which may be made of a magnetic substance. In other words, the electromagnet 510 is arranged at an end of the seating groove 143 of the socket part 140 while being arranged at a position facing an end of the seating member 232, as shown in FIG. 5. The electromagnet 510 pulls the seating member 232, which may be a magnetic substance, by electromagnetic force generated due to application of current, such that the connector 230 is mechanically fixed to the main body 100.

Here, the electromagnet 510 may be arranged throughout or at a portion of the end of the seating groove 143 in the socket part 140.

Alternatively, the electromagnet 510 may be arranged at an entrance of the seating groove 143 of the socket part 140 while being arranged at a position facing a side surface of the seating member 232, as shown in FIG. 6. The electromagnet 510 pulls the seating member 232, which is the magnetic substance, by electromagnetic force generated due to application of current, such that the connector 230 is mechanically fixed to the main body 100.

Here, the electromagnet 510 may be arranged throughout or at a portion of the entrance of the seating groove 143 in the socket part 140.

As shown in FIGS. 5 and 6, the fixing member and the detection part 340 may be arranged at different positions with respect to the seating groove 143. Alternatively, the fixing member and the detection part 340 may also be arranged at the same position.

In addition, the fixing member further includes a current supply part (not shown) to apply current to the electromagnet 510.

When the connection pins 236 are inserted into the socket grooves 141, the seating member 232, which is the magnetic substance, is strongly and mechanically connected to the electromagnet 510 by attraction force (electromagnetic force) generated due to the application of a current to the electromagnet 510. Consequently, the electromagnetic force connecting the connector 230 and the socket part 140 may be further enhanced.

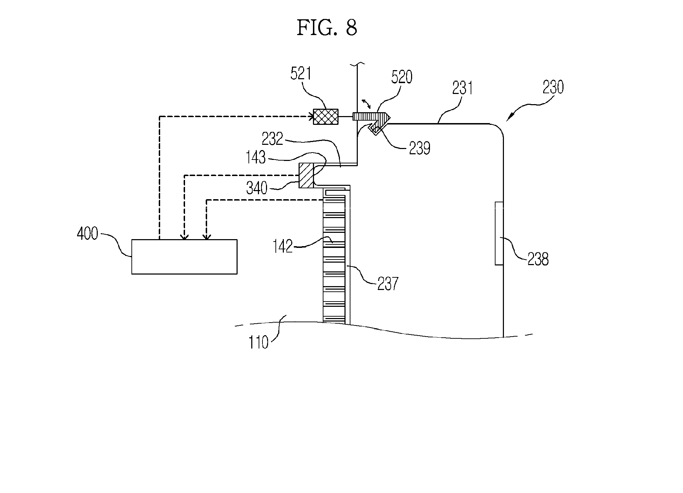

As shown in FIGS. 7 and 8, the fixing member alternatively includes a movable hook 520, a movement part 521 to move the hook 520, and a fixing groove 239 at which the hook 520 is seated.

The hook 520 and the movement part 521, which are the fixing member, are arranged at the socket part 140, whereas the fixing groove 239 is arranged at the connector 230.

The movement part 521 pulls the hook 520 based on a control command of the controller 400, thereby mechanically separating the connector 230.

That is, when the control command is input via the input part 238, the controller 400 controls the movement part 521, for example, to pull the hook 520 for a predetermined time from when the command is input.

As shown in FIG. 7, the fixing groove 239 is formed at the side surface of the seating member 232, whereas the hook 520 is arranged at the entrance of the seating groove 143 of the socket part 140 while being arranged at a position facing the fixing groove 239 of the seating member 232.

Thus, the hook 520 is pushed inward with respect to the socket part 140 when the seating member 232 slides into the seating groove 143. In this case, when the seating member 232 is seated at the seating groove 143, the hook 520 is located at the fixing groove 239 formed at the seating member 232, such that the connector 230 is mechanically fixed to the main body 100.

Alternatively, the fixing groove 239 is arranged at the housing 231 of the connector 230 while positioned to be in contact with the body part 110 of the main body 100, whereas the hook 520 is arranged in the vicinity of the seating groove 143 of the socket part 140 in the body part 110 while being arranged at a position facing the fixing groove 239 of the seating member 232, as shown in FIG. 8.

Thus, when the seating member 232 is slid into the seating groove 143, the hook 520 is pushed by a predetermined distance depending on, for example, the distance to an end of the housing 231. In this case, when the seating member 232 is seated at the seating groove 143, the hook 520 is located at the fixing groove 239 formed at the housing 231, such that the connector 230 is mechanically fixed to the main body 100.

Alternatively, at least one hook 520, movement part 521, and fixing groove 239 may be provided.

When the seating member 232 is seated at the seating groove 143, the connector 230 is mechanically fixed to the main body 100 by the hook 520 and the fixing groove 239. Consequently, the connection force between the connector 230 and the socket part 140 may be further enhanced.

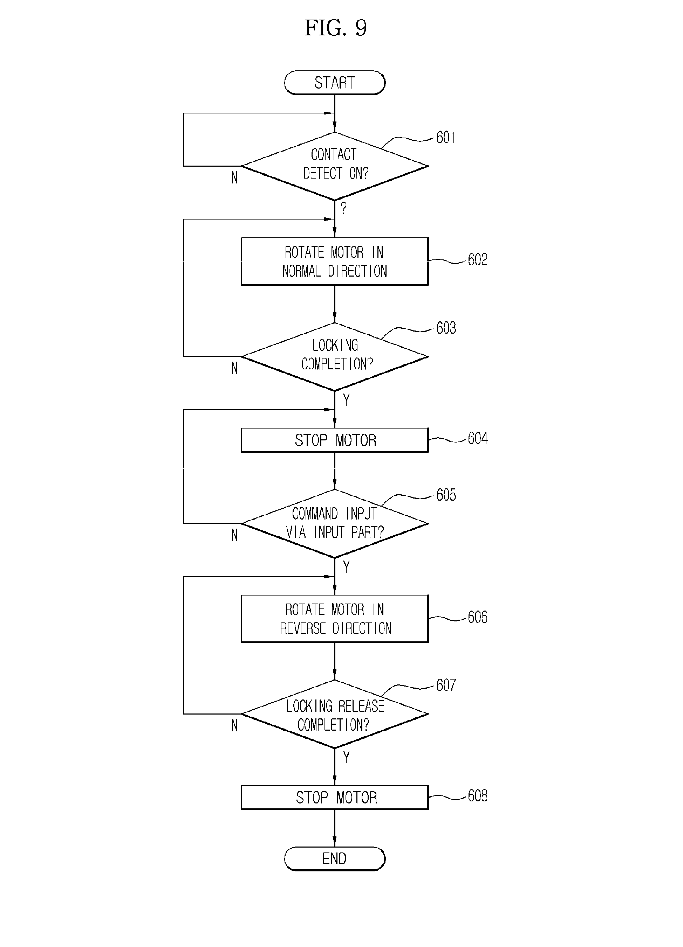

FIG. 9 is an exemplary process flowchart for controlling the ultrasound diagnostic apparatus described herein. For purposes of discussion, the process flowchart of FIG. 9 will be described with reference to the exemplary ultrasound diagnostic apparatuses of FIGS. 1 to 3, as described above, but is not intended to be limited thereto. In addition, for purposes of discussion and the examples illustrated in FIGS. 9-14, a rotation direction for locking will be referred to as "normal rotation" and a rotation direction for releasing the lock will be referred to as "reverse rotation."

First, the ultrasound diagnostic apparatus detects contact between the connector 230 and the main body 100 through the detection part 340 (at step 601), and the motor 320 is rotated in a normal direction when contact between the connector 230 and the main body 100 is detected (at step 602).

In this case, the coupling member 310 is rotated by rotation of the motor 320, and the shaft 233 of the connector 230 coupled to the coupling member 310 is rotated by the rotation of the coupling member 310.

Also, when contact between the connector 230 and the main body 100 is detected, the connector 230 is primarily and mechanically fixed to the main body 100 through the fixing member 510 or 520.

In addition, when the fixing member is the electromagnet 510, the ultrasound diagnostic apparatus applies current to the electromagnet 510.

The ultrasound diagnostic apparatus determines whether or not locking between the connector 230 and the main body 100 is completed during normal rotation of the motor 320 (at step 603).

Here, the determining whether or not locking is completed includes determining whether or not the number of normal rotations of the motor 320 is the predetermined number of rotations or more during the normal rotation of the motor 320. Furthermore, the determining whether or not locking is completed includes determining whether or not a normal rotation time of the motor 320 is the predetermined time, or whether or not current of the motor 320 is the predetermined current or more.

That is, when the motor 320 is rotated in the normal direction by the predetermined number of rotations or the current of the motor 320 is the predetermined current or more, it is determined that the locking is completed.

The locking is completed when the locking member 234 formed at the shaft 233 is seated at the locking groove 330 in the main body 100. The connector 230 is secondarily and mechanically connected to main body 100 by such locking, and the connection pins 236 of the connector 230 come into contact with the socket pins of the socket 142, such that the connector 230 is electrically fixed to the main body 100.

When locking between the connector 230 and the main body 100 is completed, the ultrasound diagnostic apparatus stops the motor 320 (at step 604).

Subsequently, the ultrasound diagnostic apparatus determines whether or not a command is input via the input part 238 (at step 605).

In this case, when the input part 238 is arranged at the connector 230, the signal generated upon pressing the input part 238 is transmitted to the controller 400 through any one of the connection pins 236.

When the command is input via the input part 238, the ultrasound diagnostic apparatus rotates the motor 320 in a reverse direction (at step 606).

In this case, the coupling member 310 is rotated in reverse by the reverse rotation of the motor 320, the shaft 233 coupled to the coupling member 310 is rotated in reverse by the reverse rotation of the coupling member 310, and the locking member 234 is separated from the locking grove 330 by the reverse rotation of the shaft 233.

Also, when the command is input via the input part 238, the ultrasound diagnostic apparatus controls the fixing member 510 or 520 so that the connector 230 is primarily and mechanically separated from the main body 100.

When the fixing member is the electromagnet 510, the ultrasound diagnostic apparatus blocks the current applied to the electromagnet 510, whereas when the fixing member is the hook 520, the ultrasound diagnostic apparatus controls the movement part 521 to move the hook 520.

Subsequently, the ultrasound diagnostic apparatus determines whether or not locking release between the connector 230 and the main body 100 is completed (at step 607).

Here, the determining whether or not locking release is completed includes determining whether or not the number of reverse rotations of the motor 320 is the predetermined number of rotations or whether or not a reverse rotation time of the motor 320 is the predetermined time.

Thereafter, when locking release is completed, the ultrasound diagnostic apparatus stops the motor 320 (at step 608).

Accordingly, the connector 230 of the associated probe 200 may be mechanically and electrically coupled to the main body 100 with ease using the motor 320.

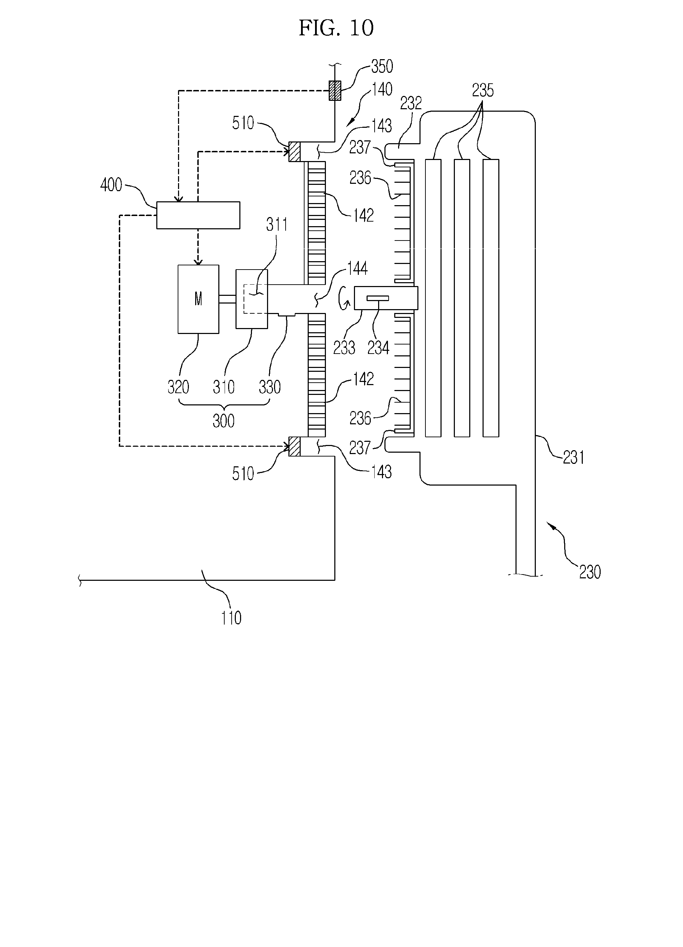

FIG. 10 illustrates a view of an example ultrasound diagnostic apparatus. As shown in FIG. 10, the ultrasound diagnostic apparatus includes a main body 100, probes 200, a coupling assembly 300, and a controller 400.

Since configurations of the main body 100 and the probes 200 are similar to those described above with respect to FIGS. 1-2, no description will be given thereof. In the present embodiment, a connector 230 of each probe 200 is not provided with the input part of the above embodiment.

The coupling assembly 300 includes a coupling member 310 coupled to the end of the shaft 233 of the connector 230 of the associated probe 200 and rotated by interlocking with the shaft 233 while being arranged at the position extending from the insertion hole 144 of the socket part 140, a motor 320 to apply rotation force to the coupling member 310 so as to drive the shaft 233, a locking groove 330 to seat the locking member 234 of the shaft 233, and an input part 350 used to input a command by a user while being arranged at the body part 110.

Here, the coupling member 310 further includes a coupling groove 311 to which the end of the shaft 233 is inserted and coupled. The coupling groove 311 has a shape equal to the sectional shape of the shaft 233, and the locking groove 330 serves to lock the connector 230 into the main body 100 during seating of the locking member 234.

The input part 350 is constituted of buttons, etc. When the input part 350 is pressed by a user, a signal generated upon pressing the input part 350 is transmitted to the PCB 235 electrically connected to the input part 350.

The controller 400 is electrically connected to the motor 320. The controller 400 transmits a drive control signal to the motor 320 so as to execute automatic locking between the main body 100 and the connector 230 based on a command input by a user via the input part 350. On the other hand, the controller 400 transmits a drive control signal to the motor 320 so as to automatically release locking between the main body 100 and the connector 230 when the command signal is again input via the input part 350.

Here, locking between the main body 100 and the connector 230 refers to a case in which the locking member 234 of the shaft 233 is seated at the locking groove 330 of the coupling assembly 300 by rotation of the shaft 233. As such, the connector 230 is mechanically fixed to the main body 100 and simultaneously the connection pins 236 of the connector 230 come into contact with the socket pins of the socket part 140. As a result, the connector 230 is electrically connected to the main body 100.

The ultrasound diagnostic apparatus in the example illustrated in FIG. 10 further includes a fixing member such as that shown in FIGS. 5 to 8 and as described above.

The controller 400 is electrically connected to the fixing member 510. When a command is input via the input part 350, the controller 400 controls the fixing member 510 so that the connector 230 is mechanically connected to the main body 100. On the other hand, when the command signal is again input via the input part 350 in the locked state, the controller 400 controls the fixing member 510 so that the connector 230 is mechanically separated from the main body 100. Thus, the controller 400 transmits a drive control signal to the motor 320 so as to automatically release locking between the main body 100 and the connector 230.

That is, when the fixing member is an electromagnet, the ultrasound diagnostic apparatus applies current to the electromagnet for locking and blocks the current applied to the electromagnet for releasing the lock. Alternatively, when the fixing member is a hook, the ultrasound diagnostic apparatus moves the hook during the locking release.

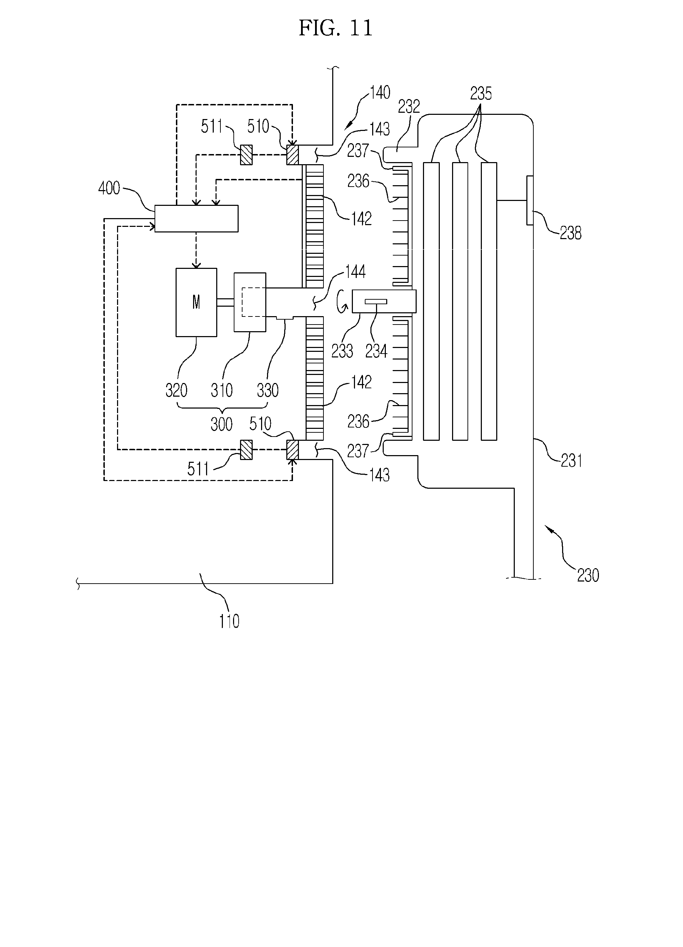

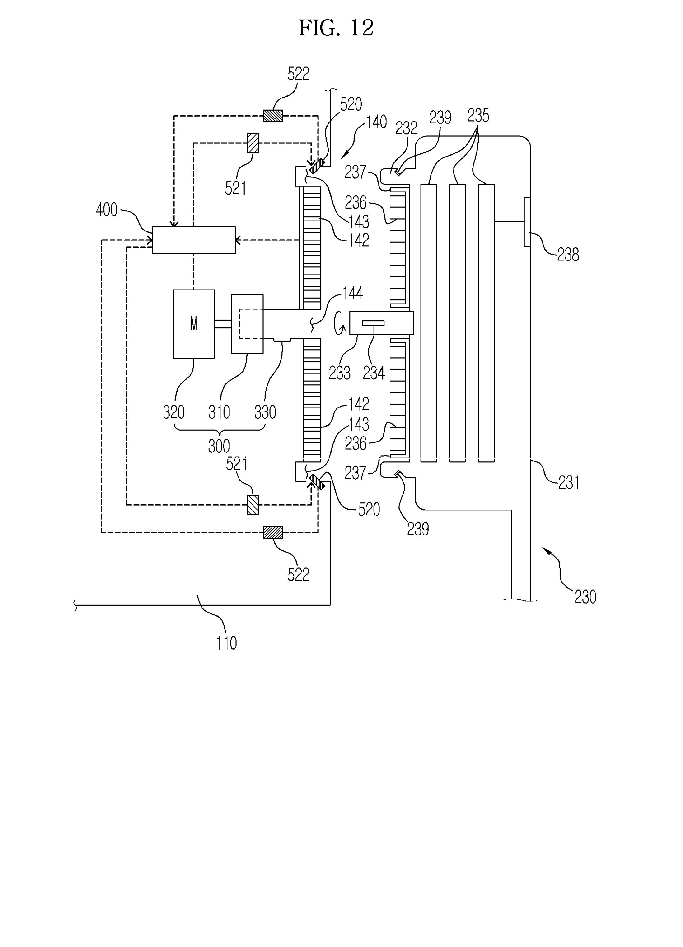

FIGS. 11 and 12 are exemplary views illustrating another example of an ultrasound diagnostic apparatus.

FIG. 11 is an exemplary view illustrating the ultrasound diagnostic apparatus including an electromagnet as a fixing member. FIG. 12 is an exemplary view illustrating the ultrasound diagnostic apparatus including a hook as the fixing member.

The ultrasound diagnostic apparatus according to the present illustrated embodiment includes a main body 100, probes 200, a coupling assembly 300, a controller 400, and a fixing member 510 or 520. Since configurations of the main body 100 and the probes 200 are similar to those of the examples described above, no additional description will be provided for this example.

As shown in FIG. 11, the coupling assembly 300 includes a coupling member 310 coupled to the end of the shaft 233 of the connector 230 of the associated probe 200 and rotated by interlocking with the shaft 233 while being arranged at a position extending from the insertion hole 144 of the socket part 140, a motor 320 to apply rotation force to the coupling member 310 so as to drive the shaft 233, and a locking groove 330 to seat the locking member 234 of the shaft 233.

The fixing member includes an electromagnet 510 to generate electromagnetic force using current, and a magnetic field detection part 511 to detect a magnetic field of the electromagnet 510. The electromagnet 510 may be installed, for example, in the vicinity of the seating groove 143.

Current is applied to the electromagnet 510 in order to generate electromagnetic force when the connector 230 is coupled to the main body 100, whereas the current is blocked from the electromagnet 510 to remove the electromagnetic force when the connector 230 is mechanically separated from the main body 100.

That is, the controller 400 continuously applies current to the electromagnet 510, and controls rotation of the motor 320 in the normal direction to lock the connector 230 into the main body 100 when a change in the magnetic field is detected by the magnetic field detection part 511. On the other hand, when a command is input via the input part 238, the controller 400 blocks the current applied to the electromagnet 510 for a predetermined time to mechanically separate the connector 230 from the main body 100, and controls rotation of the motor 320 in the reverse direction to automatically release the lock between the connector 230 and the main body 100.

In an example, the main body 100 may be locked to the connector 230 when, for example, the locking member 234 of the shaft 233 is seated at the locking groove 330 of the coupling assembly 300 when the shaft 233 is rotated in an appropriate direction. As such, the connector 230 is mechanically fixed to the main body 100 and the connection pins 236 of the connector 230 simultaneously come into contact with the socket pins of the socket part 140. As a result, the connector 230 is electrically and mechanically connected to the main body 100.

In addition, the fixing member further includes a current supply part (not shown) to apply current to the electromagnet 510.

Thus, the seating member 232, which is the magnetic substance, is strongly and mechanically connected to the electromagnet 510 by attraction force (electromagnetic force) generated due to application of current to the electromagnet 510. Consequently, the electromagnet force connecting the connector 230 and the socket part 140 may be further enhanced. Furthermore, the motor 320 may be controlled based on the change in the magnetic field generated by the electromagnet 510, thereby automatically performing locking between the connector 230 and the main body 100.

As shown in FIG. 12, the coupling assembly 300 includes a coupling member 310 coupled to the end of the shaft 233 of the connector 230 of the associated probe 200. The coupling member 310 may be rotated by interlocking with the shaft 233 while being arranged at the position extending from the insertion hole 144 of the socket part 140. The coupling assembly 300 also includes a motor 320 to apply rotation force to the coupling member 310 so as to drive the shaft 233 and a locking groove 330 to seat the locking member 234 of the shaft 233.

The fixing member includes a movable hook 520, a movement part 521 to automatically move the hook 520, and a position detection part 522 to detect position change of the hook 520.

The hook 520 is moved toward the body part 110 by elasticity generated when the seating member 232 of the connector 230 is slid into the seating groove 143 of the socket part 140. Subsequently, when the hook 520 is located at the fixing groove 239, the hook 520 is seated in the fixing groove 239, and thus the connector 230 is mechanically connected to the main body 100.

In this case, the position detection part 522 detects a position of the hook 520 and transmits the detected position to the controller 400.

When a change in the position of the hook 520 is detected, the controller 400 controls rotation of the motor 320 in the normal direction to lock the connector 230 into the main body 100. On the other hand, when a command is input via the input part 238, the controller 400 controls driving of the movement part 521 for a predetermined time to mechanically separate the connector 230 from the main body 100, moves the hook 520 toward the body part 110, and controls rotation of the motor 320 in the reverse direction to automatically release locking between the connector 230 and the main body 100.

Here, locking between the main body 100 and the connector 230 refers to a case in which the locking member 234 of the shaft 233 becomes seated at the locking groove 330 of the coupling assembly 300 through proper rotation of the shaft 233. As such, the connector 230 is mechanically fixed to the main body 100 and simultaneously the connection pins 236 of the connector 230 come into contact with the socket pins of the socket part 140. As a result, the connector 230 is electrically and mechanically connected to the main body 100.

Alternatively, at least one hook 520, movement part 521, and fixing groove 239 may be provided.

Thus, the seating member 232, which is the magnetic substance, is strongly and mechanically connected to the electromagnet 510 by attraction force (electromagnetic force) generated due to application of current to the electromagnet 510. Consequently, connection force between the connector 230 and the socket part 140 may be further enhanced. Furthermore, driving of the motor 320 is controlled based on the magnetic field change of the electromagnet 510, thereby automatically performing locking between the connector 230 and the main body 100.

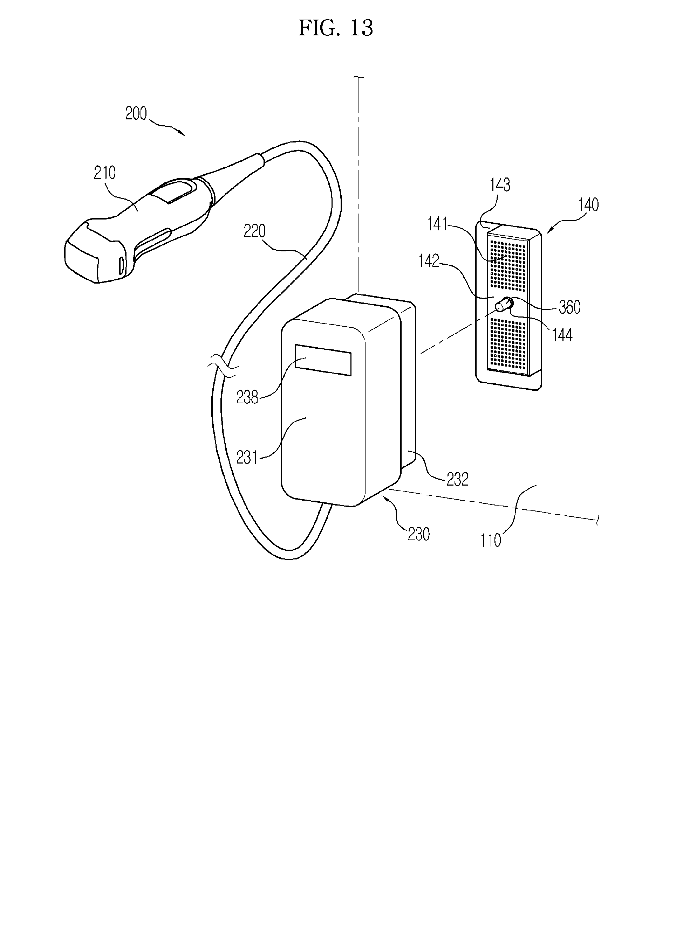

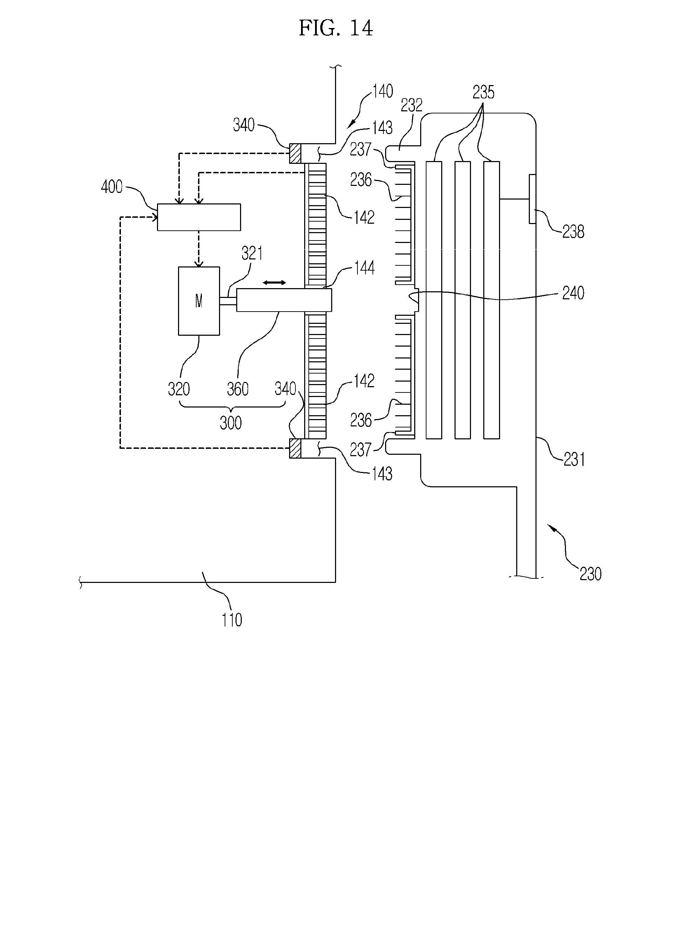

FIGS. 13 and 14 are views illustrating yet another exemplary ultrasound diagnostic apparatus according to yet another exemplary embodiment. Components similar to the above illustrated embodiment will be described in brief.

As described above and shown in FIG. 1, the ultrasound diagnostic apparatus includes a main body 100 and probes 200. Referring back to FIG. 1, the main body 100 includes a body part 110, a display part 120, a manipulation part 130, a control unit (not shown), and a socket part 140.

In the example illustrated in FIG. 13, the socket part 140 includes a socket 142 having socket grooves 141 into which connection pins of a connector 230 of each probe 200 are inserted during connection of the connector 230 of the probe 200, a seating groove 143 formed at an outer peripheral surface of the socket 142, to seat the connector 230 of the probe 200, and an insertion hole 144 into which a shaft 360 is inserted while being formed at a predetermined region of the socket 142.

When the connector 230 of the associated probe 200 is connected to the socket part 140 of the main body 100, a probing part 210 of the probe 200 is electrically connected to various components of the main body 100. Thus, the main body 100 may control various components of the probe 200, thereby activating the probe 200. In addition, the main body 100 may process or display various information acquired by the probe 200.

A plurality of socket parts 140 may be provided, and the probes 200 having characteristics different from one another may be selectively connected to the plural socket parts 140.

Each probe 200 refers to a component to directly execute ultrasound diagnosis upon an examination portion of an object to be examined. The probe 200 is connected to the main body 100 executes ultrasound diagnosis based on a command transmitted from the main body 100, and transmits the diagnosed ultrasound signal to the main body 100.

Each probe 200 includes a probing part 210, a cable 220, and a connector 230.

As shown in FIGS. 13 and 14, the connector 230 includes a housing 231, a seating member 232 formed to protrude from a flange portion of one surface of the housing 231, and a shaft hole 240 recessed inward of the housing 231.

The shaft hole 240 has a polygonal shape or an oval shape in section so as to be stably locked into a shaft 360.

In addition, the connector 230 further includes at least one printed circuit board (PCB) 235, connection pins 236 moved depending on rotation of the shaft 360 so as to electrically connect the associated probe 200 to the main body 100, a terminal 237, and an input part 238 used to input a command by a user.

The PCB 235 electrically connected to the input part 238 transmits the signal of the input part 238 to the main body 100 through any one of the connection pins 236. Thus, the connector 230 is mechanically and electrically separated from the main body 100.

In order to enhance user convenience, the socket part 140 of the main body 100 should be mechanically and electrically coupled to the connector 230 of the probe 200 so as to allow coupling to each other to be secure and easy.

The ultrasound diagnostic apparatus further includes, but is not limited to, a coupling assembly 300 and a controller 400.

The coupling assembly 300 and the controller 400 may be provided within the body part 110 of the main body 100 or alternatively, within the housing 231 of the connector 230 of the associated probe 200. For purposes of the example illustrated in FIGS. 13 and 14, the coupling assembly 300 and the controller 400 will be described as being arranged within the body part 110 of the main body 100. However, the ultrasound diagnostic apparatus and techniques described herein are not intended to be limited thereto.

As shown in FIG. 14, the coupling assembly 300 includes a shaft 360 inserted into the shaft hole 240 of the connector 230, a motor 320 to apply rotation force to the shaft 360 so as to drive the shaft 360, a protrusion member 321 connected to the motor 320 so as to apply rotational force to the shaft 360 while outwardly protruding the shaft 360 during contact of the connector 230, and a detection part 340 to detect contact between the socket part 140 and the connector 230.

For example, when contact between the socket part 140 and the connector 230 is detected, the shaft 360 outwardly protrudes through the insertion hole 144 of the socket part 140, whereas when a command is input via the input part 238 by a user, the shaft 360 is retracted into the insertion hole 144 of the socket part 140.

As shown in FIG. 14, the detection part 340 may be located within the seating groove 143. In this example, when the seating member 232 of the connector 230 comes into contact with the seating groove 143, for example, when the seating member 232 is inserted into the seating groove 143, the detection part 340 detects contact between the seating member 232 and the seating groove 143.

The controller 400 is electrically connected to the motor 320. When contact between the main body 100 and the connector 230 is detected by the detection part 340, the controller 400 controls the driving of the protrusion member 321 so that the shaft 360 protrudes outwards. Consequently, the protruding shaft 360 is inserted into the shaft hole 240 of the connector 230.

The controller 400 transmits a drive control signal to the motor 320 so as to electrically connect the connector 230 to the main body 100, thereby rotating the shaft 360.

The controller 400 transmits a drive control signal to the motor 320 when a signal is transmitted from the input part 238, thereby retracting the shaft 360. Consequently, the electrical connection between the main body 100 and the connector 230 is released.

The ultrasound diagnostic apparatus in this example further includes a fixing member capable of enhancing connection force between the connector 230 of the associated probe 200 and the socket part 140 of the main body 100.

The fixing member may be provided in at least one of the main body 100 and the connector 230 of the probe 200. In the illustrated embodiment, the fixing member arranged at the main body 100 may be implemented in a variety of ways according to the exemplary configurations of the ultrasound diagnostic apparatus described herein or as desired for a particular implementation.

The automatic locking of the main body and the probe portions of the ultrasound diagnostic apparatus described herein enhances user convenience since the user no longer has to perform the manual steps associated with conventional ultrasound apparatuses.

Also, since it is unnecessary to install a locking handle for manual locking between a main body and a probe, a connector of the probe may have a simplified interior structure and the probe may have an improved design.

In addition, a shaft rotated by a locking handle does not pass through a printed circuit board within a connector, thereby enabling easy manufacture of the printed circuit board, and X-talk characteristics may be improved by insuring an interior space of the connector.

Although a few exemplary implementations of the present disclosure have been shown and described herein, it would be apparent that changes may be made as desired for a particular implementation, without departing from the principles and spirit of the subject technology described herein, the scope of which is defined in the claims and their equivalents.

## Claims

1. An ultrasound diagnostic apparatus comprising:
a probe having a connector with a shaft and a probing part connected to the connector through a cable, wherein the probing part is used to irradiate ultrasound waves into an object to be examined and to receive the ultrasound waves reflected from the object;
a main body electrically and mechanically connected to the probe and configured to generate an image of the object based on the reflected ultrasound waves received by the probing part of the probe;
a coupling assembly to detect contact between the main body and the connector and to fix the connector to the main body by automatically driving the shaft of the probe's connector; and
a controller to control driving of the coupling assembly when the contact between the main body and the connector is detected by the coupling assembly.

2. The ultrasound diagnostic apparatus of claim 1, wherein the coupling assembly comprises:
a coupling member coupled to the shaft;
a motor to rotate the coupling member;
a locking groove to seat a locking member of the shaft during rotation of the shaft; and
a detection part to detect the contact between the main body and the connector.

3. The ultrasound diagnostic apparatus according to claim 2, wherein the detection part comprises at least one of an infrared detection part, a radio frequency (RF) detection part, a switch part, and a pressure detection part.

4. The ultrasound diagnostic apparatus according to claim 2, wherein the controller is configured to control rotation of the motor in a first direction so that the locking member is seated at the locking groove, when the contact between the main body and the connector is detected by the detection part of the coupling assembly.

5. The ultrasound diagnostic apparatus according to claim 4, further comprising an input part used to receive a command input by a user,
wherein the controller is configured to control the rotation of the motor in a second direction opposite to the first direction so that the locking member of the shaft is separated from the locking groove of the coupling assembly, based on the command received via the input part.

6. The ultrasound diagnostic apparatus according to claim 1, further comprising a fixing member to mechanically fix the probe to the main body.

7. The ultrasound diagnostic apparatus according to claim 6, wherein the fixing member is an electromagnet.

8. The ultrasound diagnostic apparatus according to claim 7, further comprising an input part used to input a command by a user,
wherein the controller is configured to block a current applied to the electromagnet so that the probe is separated from the main body, based on the command received via the input part.

9. The ultrasound diagnostic apparatus according to claim 8, wherein the controller is configured to allow the current to be applied to the electromagnet, when the contact between the main body and the connector is detected by the detection part of the coupling assembly.

10. The ultrasound diagnostic apparatus according to claim 6, wherein the fixing member is a hook.

11. The ultrasound diagnostic apparatus according to claim 10, further comprising an input part used to input a command by a user,
wherein the controller is configured to control movement of the hook so that the probe is separated from the main body, based on the command received via the input part.

12. The ultrasound diagnostic apparatus according to claim 2, wherein:
the shaft has a polygonal shape or an oval shape in section; and
the coupling member has, therein, a coupling groove formed in a shape corresponding to a sectional shape of the shaft.

13. The ultrasound diagnostic apparatus according to claim 1, wherein the connector comprises a housing, a printed circuit board arranged within the housing to drive the probing part, a terminal having a plurality of pins electrically connected to the printed circuit board, and the shaft is formed to outwardly protrude from an inner portion of the housing so as to move positions of the pins in the terminal.

14. The ultrasound diagnostic apparatus according to claim 13, wherein the controller controls driving of the shaft so that the positions of the pins in the connector are moved for establishing an electrical connection between the main body and the probe.

15. A method for controlling an ultrasound diagnostic apparatus having a main body and a probe so as to enable the probe to be connected to and separated from the main body through a connector of the probe, the method comprising steps of:
detecting contact between the main body and the connector through a detection part;
automatically rotating a shaft arranged at the probe using a motor when the contact is detected;
determining whether or not a locking between the main body and the connector is completed; and
stopping the motor when the locking between the main body and the connector is determined to be completed.
